Europäisches Patentamt

European Patent Office

Office européen des brevets

(19)

(11) Veröffentlichungsnummer: **0 142 628**
**B1**

(12) **EUROPÄISCHE PATENTSCHRIFT**

(45) Veröffentlichungstag der Patentschrift:
**16.09.87**

(51) Int. Cl.⁴: **C 07 D 251/64**

(21) Anmeldenummer: **84109628.2**

(22) Anmeldetag: **13.08.84**

(54) Verfahren zur Herstellung von methylolierten veretherten Melaminen.

(30) Priorität: **26.08.83 AT 3053/83**

(43) Veröffentlichungstag der Anmeldung:
**29.05.85 Patentblatt 85/22**

(45) Bekanntmachung des Hinweises auf die Patenterteilung:
**16.09.87 Patentblatt 87/38**

(84) Benannte Vertragsstaaten:
**CH DE IT LI**

(56) Entgegenhaltungen:
**DE - A - 2 716 006**
**DE - A - 2 839 713**

(73) Patentinhaber: **Krems-Chemie Gesellschaft m.b.H.,
Hafenstrasse 77, A-3500 Krems (AT)**

(72) Erfinder: **Prantz, Erhard, Dr., Schillerstrasse 13,
A-3500 Krems (AT)**

(74) Vertreter: **Mann, Volker, Dr. et al, c/o Bayer AG
Konzernverwaltung RP Patentabteilung,
D-5090 Leverkusen 1, Bayerwerk (DE)**

**Beschreibung**

Die Erfindung betrifft ein besonders einfaches und wirtschaftliches Verfahren zur Herstellung weitgehend monomerer, hochmethylolierter veretherter Melamine, die sich durch eine niedrige Viskosität auszeichnen.

Die Herstellung weitgehend monomerer, hochmethylolierter veretherter Melamine gewinnt durch die immer breitere Einsetzbarkeit solcher Produkte stetig steigende Bedeutung. So finden sie in der Lackindustrie in sog. high solid Systemen Verwendung, werden als Nassfestmittel in der Papierindustrie eingesetzt, sind zur Veredelung von Textilien und zur Herstellung von Giessharzen geeignet und werden immer häufiger in grossen Mengen in der Kautschukindustrie, z.B. als Haftvermittler bei der Herstellung von Autoreifen, verwendet (Stahlcordreifen).

Für die Lackindustrie können sie nach Modifikation des Methylolierungs- und/oder Veretherungsgrades in Reaktivität und Elastizität verändert werden und haben somit fast unbeschränkte Einsatzmöglichkeiten.

Die klassische Herstellungsmethode (Houben-Weyl, Band 14/2, S. 365 ff) geschieht in zwei Schritten, wobei zuerst das Melamin basisch oder sauer methyloliert wird und das so gewonnene Hexamethylolmelamin (HMM) getrocknet und anschliessend, wie jedem Fachmann bekannt, sauer verethert wird.

Bei diesem Verfahren fallen wegen der zwingend notwendigen Isolierung des HMM sehr hohe Fertigungskosten an (Filter, Trockner, unterschiedliche Apparate, hoher Manipulationsaufwand).

Es sind sehr viele Versuche unternommen worden, beide Schritte zu vereinigen, doch sind die bisher gefundenen Lösungen entweder sehr aufwendig und kostenintensiv oder aber es konnten nur Produkte erhalten werden, die beschränkt einsatzfähig sind, weil ihre Viskosität zu hoch und der maximal erreichbare Veretherungsgrad zu gering ist (US-PS'en 2 998 411, 3 020 255, 3 087 837).

Der kritische Schritt aller Eintopfmethoden besteht in der Methylolierung, wobei für ein brauchbares niederviskoses Produkt mehr als 5 Mol HCHO pro Triazinring gebunden sein sollten. Diese Produkte fallen aber normalerweise sehr hochviskos (gelartige Suspensionen) an, so dass die Methylolierung entweder vorzeitig unterbrochen oder die Viskosität durch Zugabe eines Lösungsmittels erniedrigt werden muss.

Im ersten Fall, d.h. beim vorzeitigen Abbruch der Methylolierung, erhält man als Endprodukt hochviskose methylveretherte Methylolmelamine, weil die übrigen freien Aminogruppen mit Methylolgruppen zu höher molekularen Harzanteilen mit sehr hohen Viskositäten kondensieren (GB-PS 674 948).

Im zweiten Fall, d.h. bei Zugabe eines inerten Lösungsmittels, muss dieses vor bzw. nach der Veretherung entfernt werden oder man ist, wenn man als Lösungsmittel Wasser verwendet, gezwungen, zur Veretherung sehr grosse Alkoholüberschüsse einzusetzen bzw. mehrfach zu verethern. D.h., man muss entweder mit einem feuergefährlichen, die Umwelt belastenden Kohlenwasserstoff als Lösungsmittel hantieren, eine Tatsache, die technisch prinzipiell durchführbar aber kostenintensiv ist, oder aber es wird durch die mehrfache Vertherung eine mehrfache Neutralisation, Abdestillation und neuerliche Ansäuerung notwendig, welche die Raum-Zeit-Ausbeute beträchtlich vermindert.

GB-PS 1 030 268 zeigt schliesslich, dass ein grosser Formaldehydüberschuss (11 Mol) in einem Eintopfverfahren zwar hochmethylolierte Produkte liefert, doch sind diese wegen ihres Formaldehydreichtums und wegen der Belastung von Abwasser und Abluft vom Umweltschutzstandpunkt unvorteilhaft.

Das Verfahren der US-PS 2 918 452, bei dem das Methylolierungsprodukt erstarrt, anschliessend gemahlen und in der Folge sauer verethert wird, ist verfahrenstechnisch sehr nachteilig und bringt nur einen sehr beschränkten Fortschritt in der Ökonomie der Herstellung solcher Produkte.

In der US-PS 3 020 255 wird schliesslich ein Verfahren beschrieben, bei dem das ausgefallene HMM im Vakuum entwässert wird (bei 40 - 50°C), wobei aber der Niederschlag nicht mehr rührbar ist und direkt (d.h. bei einmaliger Veretherung) nur hochviskose Produkte erhalten werden können, wobei überdies die Verdampfungskosten des Wassers neben dem technischen Aufwand negativ zu Buche schlagen.

Auch bei gemäss der US-PS 2 998 411 erhält man nur über zweimalige Veretherung niederviskose Produkte.

Schliesslich ist auch die Zugabe von Methanol zur Methylolierungsmischung vorgeschlagen worden (z.B. DE-OS 2 716 006), doch ergeben auch diese Verfahren höherviskose Produkte (siehe Beispiel Nr. 7) und bedeutende Schwierigkeiten bei grösseren Ansätzen (sehr niedrige Methylolierungstemperaturen ergeben schlechten Wärmeübergang und damit sehr aufwendige Wärmeabführung).

Die DE-OS 2 839 713 schlägt vor, das Melamin in 39%iger wässriger Formaldehydlösung unter Pufferung durch Natriumborat oder -carbonat mit Hilfe eines kräftigen Hakenrührers zu methylolieren, doch erhält man auch hier bei äquivalenten Einsatzmengen gegenüber dem erfindungsgemässen Verfahren weit viskosere Produkte.

Wie aus dem Folgenden ersichtlich, wurde nun ein Verfahren gefunden, das es überraschenderweise ermöglicht, auf einem apparativ einfacheren, technisch fortschrittlicheren Weg bessere, ökonomisch günstiger herzustellende und niedrigviskosere Produkte in einem Eintopfverfahren zu erhalten.

Das erfindungsgemässe Verfahren zur Herstellung weitgehend monomerer veretherter Methylolmelamine, bei dem 1 Mol Melamin mit 6 - 8 Mol Formaldehyd in Form einer 30 - 50%igen wässrigen Lösung bei einer Temperatur von 50 - 85°C methyloliert und anschliessend in Gegenwart von Methanol verethert wird, ist dadurch gekennzeichnet, dass man die Gesamtmenge des Formaldehyds vorlegt, danach

a) im ersten Schritt 50 - 80% der Gesamtmenge an Melamin während eines Zeitraums von mehr als 30 Minuten in mindestens 2 Stufen einträgt,

b) nach Beginn der Kristallisation des Hexamethylolmelamins dem Ansatz in einem weiteren Schritt die Restmenge an Melamin zufügt und anschliessend

c) in einem dritten Schritt das als sehr gut rührbarer Kristallbrei anfallende Methylolierungsprodukt in an sich bekannter Weise mit 15 - 25 Mol Methanol bei einem pH-Wert von 2,5 bis 4,5 und bei einer Temperatur von 50 - 80°C verethert.

Bei diesem Verfahren wird im 1. Schritt vorzugsweise 60 - 90% der Gesamtmenge an Melamin in 3 oder mehr Stufen über einen Zeitraum von 0,75 bis 3 Stunden eingesetzt und der 2. Teil in mindestens 2 Stufen über 0,25 bis 3 Stunden, wobei sich eine kontinuierliche Zugabe über diesen Zeitraum bewährt hat.

Das Molverhältnis Formaldehyd zu Melamin beträgt vorzugsweise 6,5 bis 7,5 : 1, doch ist auch prinzipiell ein Einsatz in einem Molverhältnis unter 7 : 1 durchführbar.

Während des Methylolierungsschrittes kann zur Beschleunigung der Reaktion der pH schrittweise erhöht werden. Die Methylolierungstemperatur beträgt vorzugsweise 60 - 70°C.

Nach der Methylolierung wird mit Methanol versetzt, wobei die Temperatur z.B. auf 50 - 60°C fällt, vorzugsweise ohne weitere Heizung oder Kühlung auf pH 2,5 - 4,5 gestellt und je nach gewünschtem Veretherungs- und/oder Polymerisationsgrad verethert. Hierdurch kann die Reaktivität, Elastizität bzw. der Glanz des in der Folge hergestellten Lackes beeinflusst werden. Die Aufarbeitung kann so erfolgen, dass man basisch stellt, eventuell filtriert und das unumgesetzte Methanol abdampft.

Beispiel 1 zeigt eine typische Umsetzung entsprechend dem erfindungsgemässen Verfahren. Es wird 40%iges Formaldehyd in siebenfachem molaren Überschuss vorgelegt und auf 70°C erhitzt. Anschliessend werden in 20 minütigem Abstand jeweils 20% der Gesamtmenge Melamin zugegeben und der pH mit Natronlauge auf 7,5 korrigiert. Nach der vierten Zugabe fällt reines, sehr sauberes HMM aus, das nach der letzten Melaminzugabe zu einem feinkristallinen gut rührbaren Kristallbrei eindickt. Nach 20 Minuten wird mit 25 Mol Methanol versetzt, wobei sich der Ansatz auf 50°C abkühlt, und mit Schwefelsäure auf pH 3,5 gestellt. Nach einer Veretherungsdauer von 30 Minuten wird mit Natronlauge auf pH 9 gestellt, je nach Weiterverwendung filtriert und abgedampft. Es entsteht ein Produkt, das als sehr wirksamer Haftvermittler in Stahlcordreifen, oder (filtriert) als ausgezeichneter niederviskoser Lackrohstoff verwendet werden kann.

Änderungen im Methylolierungsschritt (z.B. pH-Führung) ändern die Umsetzungsgeschwindigkeit und ergeben wie im Beispiel 2 geänderte Reaktivitäten bzw. Elastizitäten im hergestellten Lack, wobei aber dennoch ausgesprochen niedrige Viskositäten im Endprodukt erreicht werden.

Beispiel 3 zeigt, dass es beim erfindungsgemässen Verfahren möglich ist, das Methanolangebot im Veretherungsschritt drastisch zu senken (z.B. zur Herstellung sehr hochreaktiver Lacktypen) und dennoch niedrigviskose Produkte erhalten werden können.

Demgegenüber erhält man gemäss dem Verfahren aus DE-OS 2 839 713 bei gleichen molaren Verhältnissen (und damit gleichem Materialeinsatz) sehr hochviskose Produkte mit weit niedrigerem Methylolierungsgrad (Beispiel 4).

Das erfindungsgemässe Verfahren zeichnet sich dadurch aus, dass das HMM vor der Veretherung als so niedrigviskoser, kristalliner Brei anfällt, dass die Umsetzung in normalen Rührwerkskesseln durchgeführt werden kann, was von grossem wirtschaftlichem Nutzen ist. Demgegenüber sind kräftige Hakenrührer, wie sie DE-OS 2 839 713 vorschreibt, bei anderen Eintopfverfahren nicht zu umgehen. (Beispiel 5 und 6 zeigt die Umsetzung gemäss DE-OS 2 839 713 in normalen Rührwerkskessel.)

Beispiel 7 schliesslich zeigt, dass äquivalent niedrige molare Verhältnisse nach anderen Verfahren zu Methylolverbindungen führen, die bei gleichen Analysenmethoden weit schlechtere Methylolierungsgrade und Viskositäten zeigen.

Beispiele 8 und 9 zeigen Betriebsansätze, wie sie zur Herstellung von Lackrohstoffen und/oder Haftvermittlern durchgeführt wurden. Sie demonstrieren den Vorteil des Verfahrens und seine Flexibilität. Die HMM-Suspension ist so niedrigviskos, dass der Rührer vor der Methanolzugabe sogar abgestellt und der Ansatz anschliessend mit Methanol aufgeschlämmt und verethert werden kann. Die erhaltenen Produkte zeigten sehr gute Qualität.

Ein analoger Ansatz mit einmaliger Melaminzugabe (gemäss Beispiel 5) ist nach ca. 2 Stunden so fest geliert, dass er sich nicht mehr verethern lässt.

Aus den Ausführungsbeispielen wird ersichtlich, dass gegenüber dem Stand der Technik für dieselbe Qualität ein geringerer HCHO-Einsatz notwendig ist und damit weniger Emission bei dem Einbrennen der Lacke erreicht wird, dass eine bedeutende Einsparung im Alkoholeinsatz und damit Materialersparnis ermöglicht wird, dass eine kürzere Methylolierungszeit und damit verbesserte Raum-Zeit-Ausbeute, ein beliebiges Rührwerk und damit geringerer technischer Aufwand und dass ganz besonders die in Gegenwart und Zukunft geforderte grosse Flexibilität des Methylolierungsschrittes gegeben ist, da die Rührbarkeit der Suspension ein Vielfaches der für die Methylolierung notwendigen Zeit beträgt, die überdies durch pH bzw. Temperaturführung leicht beeinflussbar ist.

Bei gleichem Materialeinsatz, wie in DE-OS 2 839 713 beschrieben, erhält man weit bessere und niedriger-viskose Endprodukte.

Die Rührbarkeit des Kristallbreis ist weit besser als bei allen bisherigen Verfahren und das Produkt kann, auch wenn es sehr stark (lange) methyloliert werden soll, in einem normalen Rührwerkskessel bei normaler gebräuchlicher Rührgeschwindigkeit hergestellt werden, wo beim Einsatz anderer Methoden der Rührer stecken bleibt und der Einsatz kräftiger Hakenrührer oder gar ein Kneten notwendig ist.

Diese Eigenschaft ist von nicht zu unterschätzendem Wert, erleichtert sie doch die technische Verfahrensweise ungemein, weil nicht mehr die permanente Gefahr des Festwerdens des Methylolproduktes und der dann notwendigen «bergmännischen Entleerung» des Kessels besteht. Die besonders gefürchtete Bildung von Krusten am Rand des Rührwerkskessels, die nur sehr schlecht oder überhaupt nicht zu verethern sind, wird ebenso vermieden. Der Brei kann sogar längere Zeit ohne Rühren gehalten werden und lässt sich nachträglich sehr leicht im Veretherungsalkohol suspendieren und schnell verethern (Beispiel 8).

Hierdurch ist dem Fachmann die Möglichkeit gegeben, durch Veränderung der Veretherungsart-, -zeit, -temperatur, -pH, Wasserzugabe etc. das Endprodukt nach Belieben im Rahmen der durch die Methylolierung vorgegebenen Grenzen in Reaktivität, Elastizität, Glanz der Lacke oder in der Viskosität zu verändern.

Die vorliegende Erfindung ist besonders deshalb überraschend, weil es aufgrund der umfangreichen, langjährigen und international durchgeführten Versuche als unmöglich galt, so niedrigviskose Produkte ökonomisch im Eintopfverfahren herzustellen und die einschlägige Industrie grosse Investitionen in Filter, Kneter und Destillationskapazitäten für notwendig hielt.

### Beispiele

#### Beispiel 1

Melamin : Formaldehyd : Methanol        1 : 7 : 25

Verfahren gemäss Erfindung

In einem 2 l Dreihalskolben werden 527 g einer 40%igen Formaldehydlösung vorgelegt, 25,2 g Melamin zugegeben und bei 70°C anschliessend mit Natronlauge auf pH 7,5 gestellt. Nach 20 Minuten werden wieder 25,2 g Melamin zugegeben, wobei der pH gegebenenfalls auf 7,5 mit Natronlauge nachgestellt wird. Dies wird noch dreimal mit jeweils 20 min Abstand wiederholt, wobei die pH-Einstellungen 7,5, 8,5, 9 betragen und nach der vierten Zugabe kristallines Hexamethylolmelamin ausfällt. Nach der fünften Zugabe beginnt sich der Ansatz langsam einzudicken, wobei ein grobkristalliner gut rührbarer Kristallbrei entsteht.

20 Minuten nach der letzten Zugabe wird das Methanol zugegeben, wobei der Ansatz auf 50°C abkühlt und mit Schwefelsäure auf einen pH von 3,5 gestellt wird. Nach 30 Minuten ist der Ansatz ganz klar und wird mit Natronlauge auf einen pH von 9 gestellt. Über einen Filter wird das kristalline Natriumsulfat abfiltriert und der Ansatz auf 97,5% Trockensubstanz (TS) abgedampft. (Die TS wird dadurch bestimmt, dass eine Probe im Kölbchen 1 Stunde lang am Wasserstrahlvakuum bei 100°C abgedampft wird.) Man erhält ein Produkt, das bei 20°C eine Viskosität von 6,2 Ps. s. besitzt, ca. 5,8 - 5,9 Mole Methylolgruppen pro Mol Triazinring gebunden hat und ca. 5,2 Mole methylveretherte Methylolgruppen enthält.

#### Beispiel 2

Melamin : Formaldehyd : Methanol        1 : 7 : 25

Verfahren gemäss Erfindung

Ansatz wie Beispiel 1 aber Zugabezeiten und pH wie folgt:
30 Minuten/pH 7,5; 20 Minuten/pH 8; 20 Minuten/pH 8,5; 15 Minuten/pH 9; 10 Minuten/pH 9. Endprodukt: TS = 97,8, Viskosität 10,2 Pa.s. bei 20°C.

#### Beispiel 3

Melamin : Formaldehyd : Methanol        1 : 7 : 20

Verfahren gemäss Erfindung

Analog dem Beispiel 1 werden dieselben Ansatzmengen in der gleichen Zugabeweise umgesetzt, jedoch werden folgende pH-Stufen eingehalten: 7,5, 7,5, 7,5, 8, 8. Ca. 20 Minuten nach der letzten Zugabe wird der Rührer abgestellt und nach 60 Minuten 640 g Methanol zugegeben und der pH der Veretherungslösung auf 4 einstellt. Nach 30 Minuten ist der Ansatz klar, nach weiteren 30 Minuten wird auf pH 110 gestellt und wie beschrieben verfahren. Man erhält ein Produkt mit einer Viskosität von 6,7 Pa.s. bei 20°C und einen Trockengehalt von 97,6%.

#### Beispiel 4

Melamin : Formaldehyd : Methanol        1 : 7 : 20

Vergleichsversuch gemäss DE-OS 2 839 713

527 g einer 40%igen Formaldehydlösung werden mit Borax auf pH 7 eingestellt und auf 65°C aufgeheizt. Dann werden 126 g Melamin zugegeben und bei 65°C gerührt. Nach 90 Minuten ist der Ansatz so eingedickt, dass der Rührer stecken zu bleiben droht und sich eine Kruste am Rand bildet, die sich aber nach Zugabe von 640 g Methanol und Einstellen eines pH von 3,5 mit Schwefelsäure nach 75 Minuten aufgelöst hat. Aufarbeitung wie unter 1 beschrieben ergibt ein Produkt, das bei 95,1% TS eine Viskosität von 151 Pa.s. bei 20°C aufweist.

#### Beispiel 5

Melamin : Formaldehyd : Methanol        1 : 7 : 20

Vergleichsbeispiel gemäss DE-OS 2 839 713

527 g einer 40%igen Formalinlösung werden vorgelegt, mit Natronlauge auf pH 7 eingestellt und auf 65°C aufgeheizt. Anschliessend werden 126 g Melamin zugegeben, nach ca. 90 Minuten ist der Ansatz nur mehr schwer rührbar, wobei der Rührer stecken bleibt und nach weiteren 30 Minuten ist er so fest, dass er sich auch bei langem Kochen mit mit Schwefelsäure angesäuertem Methanol nicht auflösen lässt und verworfen werden muss.

#### Beispiel 6

Melamin : Formaldehyd : Methanol        1 : 7 : 20

Abänderung von Beispiel 5

Ein Ansatz wie in Beispiel 5 wird nach 90 Minuten gerade noch rührbar, wie in Beispiel 1 beschrieben verethert. Man erhält ein Produkt, dass bei 96,1 TS und 20°C eine Viskosität von 80,5 Pa.s. besitzt.

#### Beispiel 7

Verfahren gemäss DE-OS 2 716 006

527 g 40%ige Formalinlösung wird vorgelegt, mit Natronlauge auf pH 7 eingestellt anschliessend wird 126 g Melamin und 220 g Methanol zugegeben und auf 50°C erhitzt, nach 2 Stunden wird mit 420 g Methanol versetzt, mit Schwefelsäure auf pH 4 gestellt und 1 Stunde verthert. Das Produkt ist nach Aufarbeitung wie unter Beispiel 1 beschrieben bei 95% TS nicht fliessfähig.

*Beispiel 8*

Melamin : Formaldehyd : Methanol　　　1 : 7 : 24

Verfahren gemäss Erfindung

815 kg 50%ige Formalinlösung werden vorgelegt, auf 65°C erwärmt, 40 kg Melamin zugegeben und der pH mit 10%iger Natronlauge auf 7,5 eingestellt. Nach 20 Minuten werden wiederum 40 kg Melamin zugegeben und der pH wie oben nachgestellt usw. Nach der vierten Zugabe wird der pH wie oben auf 8 eingestellt, ebenso nach der fünften Zugabe. Während der Reaktion hat sich der Kessel auf 70°C erwärmt und es ist feinkristallines Hexamethylolmelamin ausgefallen.

15 Minuten nach der letzten Melaminzugabe wird der Rührer abgestellt und nach weiteren 25 Minuten, nach Zugabe von 1.190 kg Methanol, wieder eingeschalten.

Der pH der Lösung wird mit Schwefelsäure auf 3,8 eingestellt, wobei man nach 15 Minuten eine klare Lösung erhält. Das nun 60°C warme Veretherungsgemisch wird mit Natronlauge auf pH 9,5 gestellt, auf 45 - 50°C abgekühlt, vom ausgefällten Natriumsulfat befreit und eingedampft.

Man erhält ein Produkt mit einer Endviskosität von 14,3 Pa.s. bei 97,5% TS und 20°C (6,3 Pa.s. bei 95% TS).

*Beispiel 9*

Melamin : Formaldehyd : Methanol　　　1 : 7 : 24

Verfahren gemäss Erfindung

Ansatz wie in Beispiel 8, pH 7,5/7,5/7,5/8/8, Veretherung bei pH 4, 2 Stunden lang. Endprodukt: TS 97,7, Viskosität = 11,5 Pa.s./20°C.

**Patentansprüche**

1. Verfahren zur Herstellung weitgehend monomerer veretherter Methylolmelamine, wobei 1 Mol Melamin mit 6 - 8 Mol Formaldehyd in Form einer 30 - 50%igen wässrigen Lösung bei einer Temperatur von 50 - 85°C methyloliert und anschliessend in Gegenwart von Methanol verethert wird, dadurch gekennzeichnet, dass man die Gesamtmenge des wässrigen Formaldehyds vorlegt, danach

a) im ersten Schritt 50 - 80% der Gesamtmenge an Melamin während eines Zeitraumes von mehr als 30 Minuten in mindestens 2 Stufen einträgt,

b) nach Beginn der Kristallisation des Hexamethylolmelamins dem Ansatz in einem weiteren Schritt die Restmenge an Melamin zufügt und anschliessend

c) in einem dritten Schritt das Methylolierungsprodukt mit 15 - 25 Mol Methanol bei einem pH-Wert von 2,5 bis 4,5 verethert.

2) Verfahren nach Anspruch 1, dadurch gekennzeichnet, dass man im 1. Schritt 60 - 80% der Gesamtmenge Melamin einsetzt.

3. Verfahren nach Ansprüchen 1 oder 2, dadurch gekennzeichnet, dass man im 1. Schritt das Melamin in 3 oder mehr Stufen zusetzt.

4. Verfahren nach Ansprüchen 1 - 3, dadurch gekennzeichnet, dass man im 1. Schritt das Melamin in einem Zeitraum von 0,75 - 3 Stunden einträgt.

5. Verfahren nach Ansprüchen 1 - 4, dadurch gekennzeichnet, dass man im 2. Schritt das Melamin in mindestens 2 Stufen zusetzt.

6. Verfahren nach Ansprüchen 1 - 5, dadurch gekennzeichnet, dass man das Melamin im 1. und/oder 2. Schritt kontinuierlich zusetzt.

7. Verfahren nach Ansprüchen 1 - 6, dadurch gekennzeichnet, dass man Formalin in einem Molverhältnis zu Melamin von 6,5 : 1 bis 7,5 : 1 einsetzt.

8. Verfahren nach Ansprüchen 1 bis 6, dadurch gekennzeichnet, dass man Formalin in einem Molverhältnis zu Melamin von unter 7 : 1 einsetzt.

9. Verfahren nach Ansprüchen 1 bis 8, dadurch gekennzeichnet, dass der pH bei der Methylolierung schrittweise erhöht wird.

10. Verfahren nach Ansprüchen 1 bis 9, dadurch gekennzeichnet, dass die Methylolierungstemperatur zwischen 60 und 70°C beträgt.

**Claims**

1. Process for the production of substantially monomeric etherified methylol melamines, wherein 1 mol of melamine is methylolated with 6 - 8 mols of formaldehyde in the form of a 30 - 50% strength aqueous solution at a temperature of 50 - 85°C and the product is then etherified in the presence of methanol, characterised in that the total quantity of the aqueous formaldehyde is initially introduced and then

a) in the first step 50 - 80% of the total quantity of melamine is introduced in at least 2 stages during a period of more than 30 minutes,

b) after the crystallisation of the hexamethylol melamine has begun the remaining quantity of melamine is added to the mixture in a second step and then

c) in a third step the methylolation product is etherified with 15 - 25 mols of methanol at a pH value of 2.5 to 4.5.

2. Process according to claim 1, characterised in that 60 - 80% of the total quantity of melamine is used in the 1st step.

3. Process according to claims 1 or 2, characterised in that the melamine is added in 3 or more stages in the 1st step.

4. Process according to claims 1 - 3, characterised in that the melamine is introduced over a period of 0.75 - 3 hours in the 1st step.

5. Process according to claims 1 - 4, characterised in that the melamine is added in at least 2 stages in the 2nd step.

6. Process according to claims 1 - 5, characterised in that the melamine is added continuously in the 1st and/or 2nd step.

7. Process according to claims 1 - 6, characterised in that formalin is used in a molar ratio to melamine of 6.5 : 1 to 7.5 : 1.

8. Process according to claims 1 to 6, characterised in that the formalin is used in a molar ratio to melamine of below 7 : 1.

9. Process according to claims 1 to 8, characterised in that the pH is increased stepwise during the methylolation.

10. Process according to claims 1 to 9, characterised in that the methylolation temperature is between 60 and 70°C.

## Revendications

1. Procédé de production de méthylolmélamines éthérifiées largement monomériques, dans lequel 1 mole de mélamine est méthylolée avec 6 à 8 moles de formaldéhyde sous la forme d'une solution aqueuse à 30-50% à une température de 50 à 85°C, puis est éthérifiée en présence de méthanol, caractérisé en ce qu'on introduit tout d'abord la quantité totale de formaldéhyde aqueux, puis

a) on verse en une première étape 50 à 80% de la quantité totale de mélamine en un intervalle de temps de plus de 30 minutes en au moins deux étapes,

b) après le début de la cristallisation de l'hexaméthylol-mélamine, on ajoute au mélange dans une deuxième étape la quantité restante de mélamine, puis

c) au cours d'une troisième étape, on éthérifie le produit de méthylolation avec 15 à 25 moles de méthanol à un pH de 2,5 à 4,5.

2. Procédé suivant la revendication 1, caractérisé en ce qu'on utilise dans la première étape 60 à 80% de la quantité totale de mélamine.

3. Procédé suivant les revendications 1 ou 2, caractérisé en ce que, dans la première étape on ajoute la mélamine en 3 ou plus de 3 étapes.

4. Procédé suivant les revendications 1 - 3, caractérisé en ce que dans la première étape, on verse la mélamine en un intervalle de temps de 0,75 à 3 heures.

5. Procédé suivant les revendications 1 - 4, caractérisé en ce que dans la deuxième étape, on ajoute la mélamine en au moins deux étapes.

6. Procédé suivant les revendications 1 - 5, caractérisé en ce qu'on ajoute la mélamine en continu dans la première et/ou dans la deuxième étape.

7. Procédé suivant les revendications 1 - 6, caractérisé en ce qu'on utilise de la formaline dans un rapport molaire avec la mélamine de 6,5 : 1 à 7,5 : 1.

8. Procédé suivant les revendications 1 à 6, caractérisé en ce qu'on utilise de la formaline dans un rapport molaire avec la mélamine de moins de 7 : 1.

9. Procédé suivant les revendications 1 à 8, caractérisé en ce qu'on élève le pH par étapes lors de la méthylolation.

10. Procédé suivant les revendications 1 à 9, caractérisé en ce que la température de méthylolation s'élève entre 60 et 70°C.